# EUROPÄISCHE PATENTSCHRIFT

(11) **EP 0 844 998 B1**
(45) Veröffentlichungstag und Bekanntmachung des Hinweises auf die Patenterteilung: **23.04.2003**
(21) Anmeldenummer: 96927623.7
(22) Anmeldetag: 29.07.1996
(51) Int. Cl.: C07D 491/04, A01N 43/90, A01N 43/50, C07D 413/12

(54) **HALOGENBENZIMIDAZOLE UND IHRE VERWENDUNG ALS MIKROBIZIDE**
HALOBENZIMIDAZOLES AND THEIR USE AS MICROBICIDES
BENZIMIDAZOLES HALOGENES ET LEUR UTILISATION COMME MICROBICIDES

(30) Priorität: 10.08.1995 DE 19529407; 08.03.1996 DE 19609060
(43) Veröffentlichungstag der Anmeldung: 03.06.1998
(73) Patentinhaber: Bayer CropScience AG, 40789 Monheim (DE)
(72) Erfinder: ASSMANN, Lutz, D-23701 Eutin (DE); MARHOLD, Albrecht, D-51373 Leverkusen (DE); TIEMANN, Ralf, D-51375 Leverkusen (DE); STENZEL, Klaus, D-40595 Düsseldorf (DE)
(86) Internationale Anmeldenummer: EP9603334
(87) Internationale Veröffentlichungsnummer: WO97006171

(56) Entgegenhaltungen:
- EP-A- 0 545 204
- GB-A- 1 114 943
- GB-A- 1 306 098
- US-A- 3 988 465
- CHEMICAL ABSTRACTS, vol. 114, no. 15, 15.April 1991 Columbus, Ohio, US; abstract no. 143420v, M.W. MOON ET AL.: "Preparation of aminoheterocycloquinolines as central nervous system agents." Seite 785; Spalte 2; XP002019112 & WO 90 15058 A (UPJOHN CO.) 13.Dezember 1990
- JOURNAL OF ORGANIC CHEMISTRY, Bd. 49, Nr. 12, 15.Juni 1984, EASTON US, Seiten 2158-2164, XP002019110 T.M. STEVENSON ET AL.: "Defined Dimensional Alterations in Enzyme Substrates. lin-Naphthoadenine and lin-Naphthoadenosine"
- JOURNAL OF THE CHEMICAL SOCIETY, 1963, LETCHWORTH GB, Seiten 2930-2937, XP002019111 D. HARRISON ET AL.: "Some 1- and 2-Halogenobenzimidazoles."

## Beschreibung

Die vorliegende Erfindung betrifft neue Halogenbenzimidazole, ein Verfahren zu ihrer Herstellung und deren Verwendung als Mikrobizide im Pflanzenschutz und im Materialschutz.

Es ist bereits bekannt geworden, daß bestimmte Benzimidazol-Derivate fungizide Eigenschaften besitzen (vgl. DE-A 4 139 950 und EP-A 0 517 476). So lassen sich z.B. 2-Cyano-3-dimethylaminosulfonyl-6,6,7,7-tetrafluor-[1,4]dioxino[2,3-f]benzimidazol und 2-Cyano-6,6-difluor-2-methylaminosulfonyl-[1,3]dioxolo[4,5-f]benzimidazol zur Bekämpfung von Pilzen einsetzen. Die Wirksamkeit dieser Stoffe ist gut, läßt aber bei niedrigen Aufwandmengen in manchen Fällen zu wünschen übrig.

In der GB - A 1 114 943, der US - A 3 988 465 und den Artikeln in Chem. Abstr. 114, 143 420 v (1991), J. Org. Chem. 49, 2930- 2937 (1984) und J. Chem. Soc. 1963, 2930 - 2937 werden zahlreiche Berizimidazole offenbart, die an einem der beiden Stickstoffatome einen über CO gebundenen Rest enthalten. Verbindungen, in denen an der entsprechenden Stelle ein über SO₂ verknüpfter Rest vorhanden ist, werden aber nicht erwähnt.

Weiterhin sind aus der EP - A 0 545 204 fungizid wirksame 2-Cyano-benzimidazol-Derivate bekannt. Es werden aber keine Stoffe dieses Typs beschrieben, die in der 2-Position ein Halogenatom aufweisen.

Schließlich betrifft die GB - A 1 306 098 halogenierte Berizimidazole. Die StickstoffAtome tragen jedoch abgesehen von Wasserstoff keine Substituenten.

Es wurden nun neue Halogenbenzimidazole der Formel 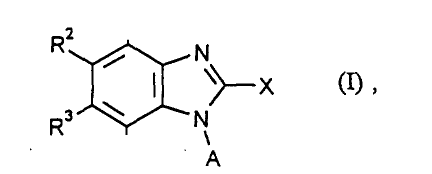 in welcher
- R²: für Wasserstoff, Fluor, Chlor oder Methoxy steht,
- R³: für Wasserstoff, Fluor, Chlor oder für -Z-R⁵ steht,
worin
Z für Sauerstoff oder Schwefel steht und
R⁵ für Phenyl steht, das einfach bis dreifach, gleichartig oder verschieden substituiert sein kann durch Fluor, Chlor, Brom, Cyano, Nitro,: Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Difluormethylsulfinyl und/oder Trifluormethylsulfonyl, oder
R⁵ für Pyrrolyl, Furyl, Thienyl, Pyrazolyl, Imidazolyl, Thiazolyl, Isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridinyl, Pyrimidinyl, Pyrazinyl oder Pyridazinyl steht, wobei jeder dieser Reste einfach oder zweifach, gleichartig oder verschieden substituiert sein kann durch Fluor, Chlor, Methyl, Methoxy, Trifluormethyl, und/oder Trifluormethoxy, oder
- R² und R³: gemeinsam für die Gruppierungen -CF₂-O-CF₂-, -O-CF₂-O-, -O-CF₂-CHF-O-, -O-CHF-CHF-O-, -O-CF₂-CF₂-O-, -O-CF₂-CFCl-Ooder -O-CFCl-CFCl-O- stehen,
- X: für Fluor, Chlor oder Brom steht und
- A: für eine Gruppierung -SO₂-R⁸ steht, worin
R⁸ für Isoxazolyl steht, wobei dieser Rest einfach oder zweifach, gleichartig oder verschieden substituiert sein kann durch Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Trifluormethylsulfinyl
oder Trifluormethylsulfonyl, Methylamino, Ethylamino, n-oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroxyminomethyl, Hydroximinoethyl, Methoxyiminomethyl, Ethoxyiminomethyl, Methoxyiminoethyl und/oder Ethoxyiminoethyl,
gefunden.

Weiterhin wurde gefunden, daß man Halogenbenzimidazole der Formel (I) erhält, wenn man
a) Benzimidazolderivate der Formel 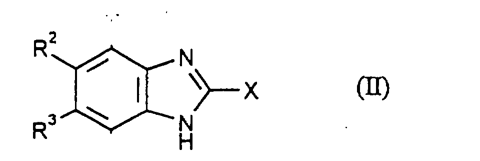 in welcher
   - R², R³: und X die oben angegebenen Bedeutungen haben, mit Halogeniden der Formel

   A-X¹ (III)
   in welcher
   - A: die oben angegebene Bedeutung hat und
   - X¹: für Chlor oder Brom steht,
   gegebenenfalls in Gegenwart eines Säurebindemittels und gegebenenfalls in Gegenwart eines Verdünnungsmittels umsetzt.

Schließlich wurde gefunden, daß die Halogenbenzimidazole der Formel (I) sehr gute mikrobizide Eigenschaften besitzen und sowohl im Pflanzenschutz als auch im Materialschutz eingesetzt werden können.

Überraschenderweise zeigen die erfindungsgemäßen Stoffe eine bessere fungizide Wirksamkeit als 2-Cyano-3-dimethyl-aminosulfonyl-6,6,7,7-tetrafluor-[1,4]-dioxino[2,3-f]benzimidazol und 2-Cyano-6,6-difluor-2-dimethylaminosulfonyl-[1,3]-dioxolo[4,5-f]benzimidazol, welches konstitutionell ähnliche, vorbekannte Wirkstoffe gleicher Wirkungsrichtung sind.

Als Beispiele für erfindungsgemäße Stoffe seien die in den folgenden Tabellen aufgeführten Halogenbenzimidazole genannt:

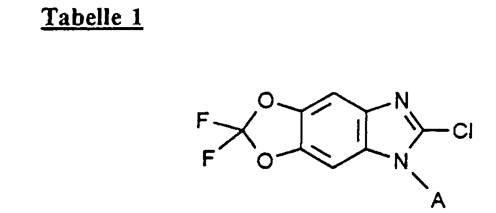

wobei A für die folgenden Substituenten steht:

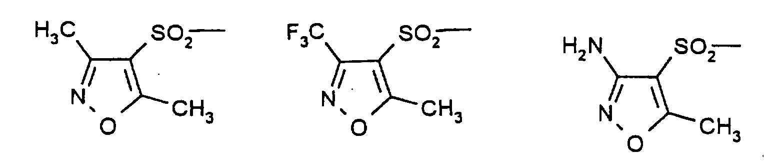

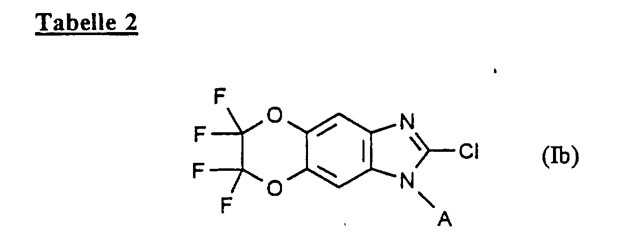

wobei A für die in Tabelle 1 genannten Substituenten steht.

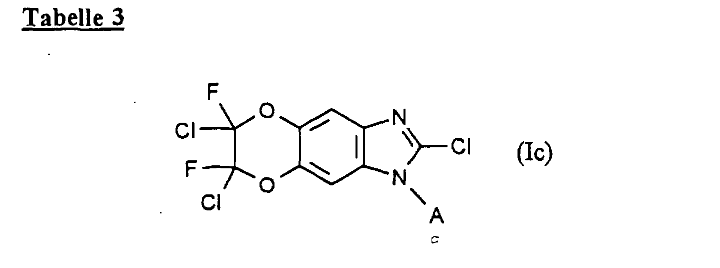

wobei A für die in Tabelle 1 genannten Substituenten steht.

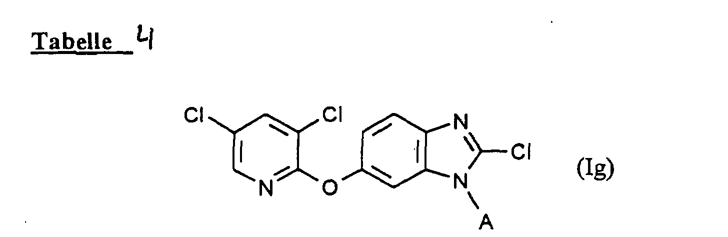

wobei A für die in Tabelle 1 genannten Substituenten steht.

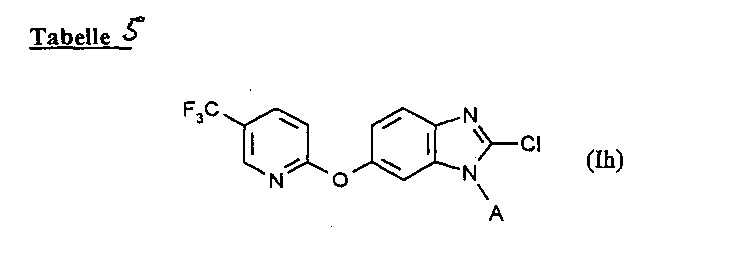

wobei A für die in Tabelle 1 genannten Substituenten steht.

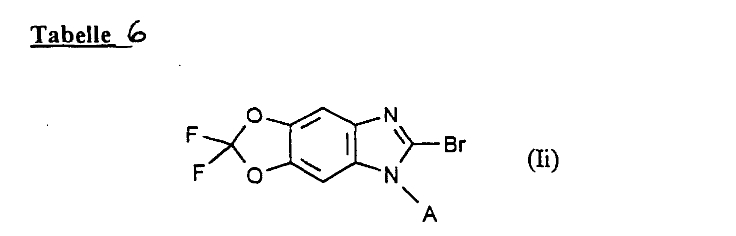

wobei A für die in Tabelle 1 genannten Substituenten steht.

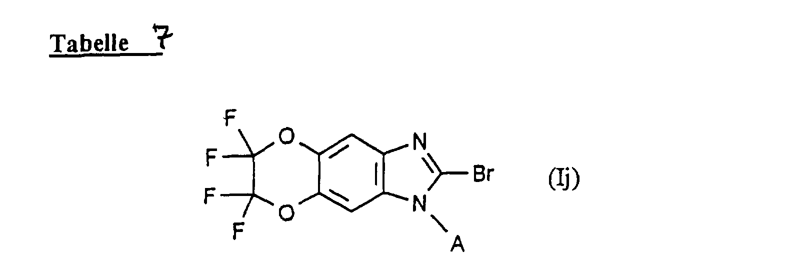

wobei A für die in Tabelle 1 genannten Substituenten steht.

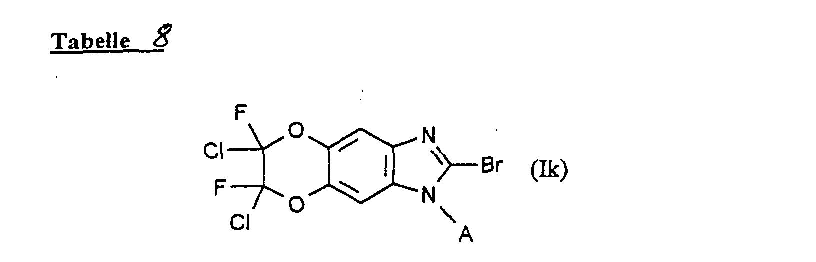

wobei A für die in Tabelle 1 genannten Substituenten steht.

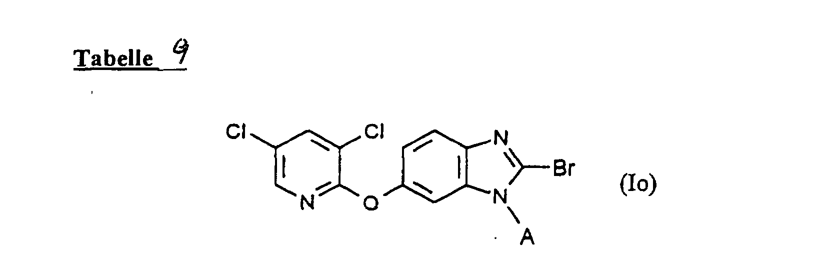

wobei A für die in Tabelle 1 genannten Substituenten steht.

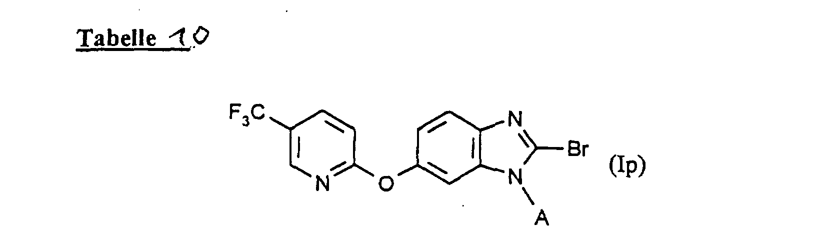

wobei A für die in Tabelle 1 genannten Substituenten steht.

Die Benzimidazolderivate der Formel (II) sind zum Teil bekannt (vergleiche Chem. Pharm. Bull. 1981, 29, 2403; Synthesis 1988, 767; J. Chem. Soc. 1922, 947; WO-A 9408456; WO-A 9207867 Bull. Soc. Chim. Fr. 1988, 1, 139-142; EP-A 308918; J. C. S. Chem. Com. 1976, 430; Liebigs Ann. Chem. 1961, 649, 114 und J. Prakt. Chem. 1965, 28, 297).

Die Benzimidazol-Derivate der Formel 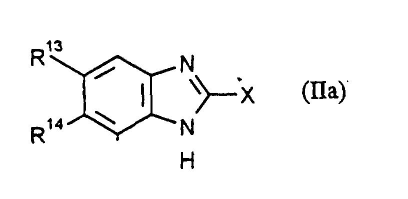 in welcher
- R¹³ und R¹⁴: unabhängig voneinander für Wasserstoff, Difluormethoxy, Trifluormethoxy, Difluorchlormethoxy, Difluorbrommethoxy, Trifluorethoxy, Difluormethylthio, Trifluormethylthio, Difluorchlormethylthio oder Difluorbrommethylthio stehen, wobei mindestens oder
R¹³ und R¹⁴ jeweils gemeinsam mit den Kohlenstoffatomen, an die sie gebunden sind,
einen einfach bis vierfach durch Fluor, Chlor und/oder Brom substituierten fünf- oder sechsgliedrigen, heterocyclischen Ring mit einem oder zwei (nicht benachbarten) Sauerstoffatomen bilden,
und
- X: für Fluor, Chlor oder Brom steht,
sind neu.

Benzimidazol-Derivate der Formel (IIa) lassen sich herstellen, indem man
(b) Benzimidazole der Formel 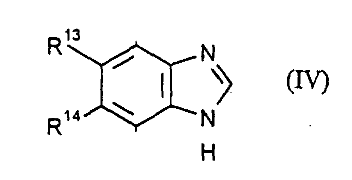 in welcher
   - R¹³ und R¹⁴: die oben angegebenen Bedeutungen haben,
   mit Halogenierungsmitteln, gegebenenfalls in Gegenwart eines Verdünnungsmittels und gegebenenfalls in Gegenwart eines Säurebindemittels umsetzt,
   oder
c) Brombenzimidazole der Formel 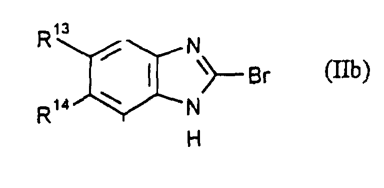 in welcher
   R¹³ und R¹⁴ die oben angegebenen Bedeutungen haben,
   mit Fluor- oder Chlor wasserstoffsäure oder einem Salz der Formel

   M⁺X²⁻ (V)

   in welcher
   - M: für ein Metalläquivalent oder ein quartäres Ammonium-, Sulfonium-, Sulfoxonium oder Phosphonium steht und
   - X²: für Fluor, oder Chlor steht,
   gegebenenfalls in Gegenwart eines Verdünnungsmittels umsetzt.

Die neuen Benzimidazolderivate sind durch die Formel (IIa) allgemein definiert.

Verwendet man 5-Trifluormethoxy-lH-benzimidazol und N-Bromsuccinimid als Ausgangsstoffe, so läßt sich der Verlauf des Verfahrens(b) durch das folgende Formelschema veranschaulichen:

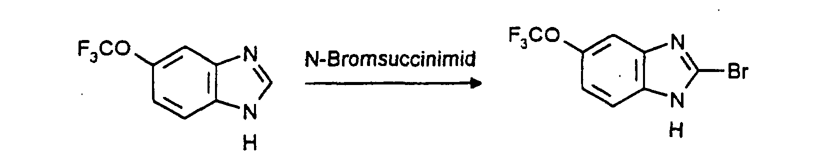

Die Benzimidazole der Formel (IV) sind teilweise bekannt, So ist zum Beispiel das 5-Pentafluorethoxy-1H-benzimidazol schon beschrieben worden (vgl. Biomed. Environ. Mass Spectrom. (1989), 18(10), 872-877).

Die Benzimidazole der Formel 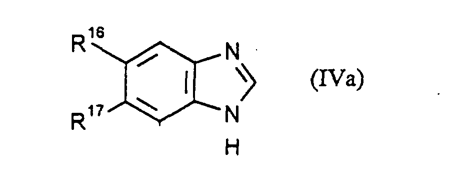 in welcher
R¹⁶ und R¹⁷ unabhängig voneinander für Wasserstoff, Difluormethoxy, Trifluormethoxy, Difluormethoxy, Difluorbrommethoxy, Trifluorethoxy, Difluormethylthio, Trifluormethylthio, Difluormethylthio oder Difluorbrommethylthio, wobei mindestens einer der Reste von Wasserstoff verschieden ist, stehen
oder R¹⁶ und R¹⁷
   gemeinsam mit den Kohlenstoffatomen and die sie gebunden sind, einen einfach bis vierfach durch Fluor, Chlor und/oder Brom substituiert fünf- oder sechsgliedrigen, heterocyclischen Ring mit einem oder zwei (nicht benachbarten) Sauerstoffatomen bilden,
sind neu.

Die Benzimidazole der Formel (IVa) lassen sich herstellen, indem man
d) Phenylendiamine der Formel 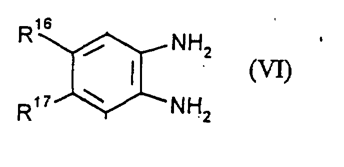
in welcher
R¹⁶ und R¹⁷ die oben angegebenen Bedeutungen haben,
mit Ameisensäure, einem ihrer Salze, oder einem ihrer Derivate, wie beispielsweise Formamid, Orthoameisensäuretrimethylester, Dialkylformamidacetate, Formamidin, s-Triazin oder Kohlenmonoxid, gegebenenfalls in Gegenwart eines Verdünnungsmittels, wie beispielsweise Wasser, Methanol, Ethanol oder Methoxyethanol, bei einer Temperatur von 0°C bis 180°C, vorzugsweise von 20°C bis 150°C, umsetzt.

Verwendet man 4-Trifluormethoxy-o-phenylendiamin und Ameisensäure als Ausgangsstoffe, so läßt sich der Verlauf des Verfahrens (d) durch das folgende Formelschema veranschaulichen:

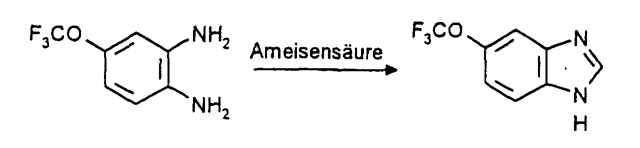

Die Phenylendiamine der Formel (VI) sind bekannt; ihre Herstellung ist z.B in DE-A 605 977, DE-A 3 621 215 oder DE-A 4 237 564 beschrieben.

Als Halogenierungsmittel kommen bei der Durchführung des Verfahrens (b) vorzugsweise elementares Halogen oder N-Halogenimide, wie N-Brom-succinimid oder N-Chlor-succinimid, in Betracht.

Als Verdünnungsmittel kommen bei der Durchführung des Verfahrens (b) alle üblichen inerten organischen Solventien in Frage. Vorzugsweise verwendbar sind aliphatische, alicyclische oder aromatischen Kohlenwasserstoffe, wie Petrolether, Hexan, Heptan, Cyclohexan, Methylcyclohexan, Benzol, Toluol, Xylol oder Decalin; ferner halogenierte Kohlenwasserstoffe, wie Chlorbenzol, Dichlorbenzol, Dichlormethan, Chloroform, Tetrachlormethan, Dichlorethan oder Trichlorethan; außerdem Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; weiterhin Nitrile, wie Acetonitril, Propionitril, n-oder i-Butyronitril oder Benzonitril, oder auch Amide, wie N,N-Dimethylformamid, N,N-Dimethylacetamid, N-Methylformanilid, N-Methylpyrrolidon und Hexamethylphosphorsäuretriamid.

Als Säurebindemittel kommen bei der Durchführung des Verfahrens (b) alle üblichen anorganischen und organischen Säureakzeptoren in Betracht. Vorzugsweise verwendbar sind Erdalkalimetall oder Alkalimetallhydride, -hydroxide, -amide, -alkoholate, -acetate, -carbonate oder -hydrogencarbonate, wie Natriumhydrid, Natriumamid, Natriummethylat, Natrium-ethylat, Kalium-tert.butylat, Natriumhydroxid, Kaliumhydroxid, Ammoniumhydroxid, Natriumacetat, Kaliumacetat, Calciumacetat, Natriumcarbonat, Kaliumcarbonat, Kaliumhydrogencarbonat, ferner Ammoniumacetat oder Ammoniumcarbonat, oder tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des Verfahrens (b) innherhalb eines größeren Bereiches variiert werden. Im allgemeinen arbeitet man bei Temperaturen zwischen -20°C und +120°C, vorzugsweise zwischen 0°C und 80°C.

Bei der Durchführung des Verfahrens (b) sowie auch bei der Durchführung der anderen Verfahren arbeitet man im allgemeinen unter Atmosphärendruck. Es ist jedoch auch möglich, unter erhöhtem Druck oder, sofern keine gasförmigen Komponenten eingesetzt werden, unter vermindertem Druck zu arbeiten.

Bei der Durchführung des Verfahrens (b) setzt man auf 1 Mol an Benzimidazol der Formel (IV) im allgemeinen eine äquivalente Menge oder auch einen Überschuß an Halogenierungsmittel ein. Die Aufarbeitung erfolgt nach üblichen Methoden.

Verwendet man 2-Brom-5-trifluormethoxy-lH-benzimidazol und Natriumchlorid als Ausgangsstoffe, so läßt sich der Verlauf des Verfahrens (c) durch das folgende Formelschema veranschaulichen:

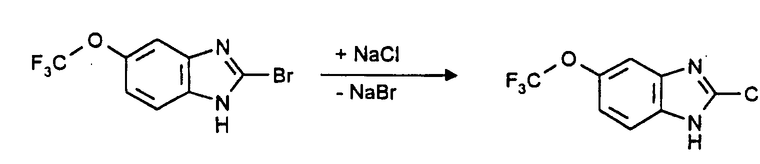

Die Verbindungen der Formel (IIb) sind Zwischenprodukte und nach dem Verfahren (b) herstellbar.

Die zur Durchführung des Verfahrens (c) weiterhin benötigte Fluor-, Chlor wasserstoffsäure sind allgemein bekannte Synthesechemikalien.

Die alternativ zur Durchführung des Verfahrens (c) als Reaktionskomponenten weiterhin benötigten Salze sind durch die Formel (V) allgemein definiert. In dieser Formel steht M vorzugsweise für ein Alkalimetall, insbesondere Lithium, Natrium oder Kalium, oder für ein quartäres Ammonium-, Sulfonium-, Sulfoxonium oder Phosphoniumion, vorzugsweise Tetraalkylammonium, mit jeweils 1 bis 12 Kohlenstoffatomen in den einzelnen Alkylketten, und X² steht für Fluor oder Chlor.

Die Salze der Formel (V) sind bekannte Synthesechemikalien.

Als Verdünnungsmittel kommen bei der Durchführung des Verfahrens (c) alle üblichen interten, organischen Solventien in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; ferner halogenierte Kohlenwasserstoffe, wie Chlorbenzol, Dichlorbenzol, Dichlormethan, Chloroform, Tetrachlormethan, Dichlorethan oder Trichlorethan; weiterhin Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; außerdem Ketone, wie Aceton, Butanon, Methyl-isobutylketon oder Cyclohexanon; ferner Nitrile, wie Acetonitril, Propionitril, n- oder i-Butyronitril oder Benzonitril; weiterhin Amide, wie N,N-Dimethylformamid, N,N-Dimethylacetamid, N-Methylformanilid, N-Methylpyrrolidon oder Hexamethylphosphorsäuretriamid; und auch Ester, wie Essigsäuremethylester oder Essigsäureethylester, sowie Sulfoxide, wie Dimethylsulfoxid und Sulfone, wie Sulfolan. Gegebenenfalls kann die Umsetzung auch in einem Zwei-Phasen-System, zum Beispiel in einem Gemisch aus Toluol und Wasser durchgeführt werden.

Auch bei der Durchführung des Verfahrens (c) können die Reaktionstemperaturen innerhalb eines größeren Bereiches variiert werden. Im allgemeinen arbeitet man bei Temperaturen zwischen -20°C und +150°C, vorzugsweise zwischen 0°C und 120°C.

Bei der Durchführung des Verfahrens (c) setzt man auf 1 mol an Brom-benzimidazol im allgemeinen eine äquivalente Menge oder auch einen Überschuß an der jeweiligen Reaktionskomponente ein. Die Aufarbeitung erfolgt nach üblichen Methoden.

Die Halogenide der Formel (III) sind bekannt und/oder können nach bekannten Verfahren hergestellt werden (J. Heterocyclic Chem. 1981, 997-1006).

Als Verdünnungsmittel zur Durchführung des erfindungsgemäßen Verfahrens (a) kommen alle inerten organischen Lösungsmittel in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol,

Dichlormethan, Chloroform, Tetrachlormethan, Dichlorethan oder Trichlorethan; Ether, wie Diethylether, Diisopropylether, Methyl-t-butylether, Methyl-tamylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Das erfindungsgemäße Verfahren (a) wird gegebenenfalls in Gegenwart eines geeigneten Säureakzeptors durchgeführt. Als solche kommen alle üblichen anorganischen oder organischen Basen infrage. Vorzugsweise verwendbar sind Erdalkalimetall- oder Alkalimetallhydride, -hydroxide, -amide, -alkoholate, -acetate, -carbonate oder -hydrogencarbonate, wie Natriumhydrid, Natriumamid, Natriummethylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, ferner Ammoniumhydroxid, Ammoniumacetat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 150°C, vorzugsweise zwischen 20°C und 120°C.

Zur Durchführung des erfindungsgemäßen Verfahrens (a) setzt man pro Mol an Benzimidazolderivat der Formel (II) im allgemeinen 1 bis 15 Mol, vorzugsweise 1 bis 2 Mol, insbesondere 1 bis 1,3 Mol Halogenid der Formel (III) ein.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Aufarbeitung erfolgt nach üblichen Methoden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide im Pflanzenschutz und auch im Materialschutz geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Krankheiten im Obst-, Gemüse- und Weinbau, wie beispielsweise gegen Phytophthora- oder Plasmopara-Arten oder zur Bekämpfung von Reis-Krankheiten, wie beispielsweise gegen den Erreger der Reisfleckenkrankheit (Pyricularia oryzae) eingesetzt werden.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe bzw. Mittel gegen Pilze, insbesondere Schimmelpilze, sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole und Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methyl-isobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 Gewichtsprozent.

Die Wirksamkeit und das Wirkungsspektrum der Wirkstoffe kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. des zusätzlichen Schutzes vor Insekten zugesetzt werden. Diese Mischungen können ein breiteres Wirkungsspektrum besitzen als die erfindungsgemäßen Verbindungen.

In vielen Fällen erhält man dabei synergistische Effekte, d.h. die Wirksamkeit der Mischung ist größer als die Wirksamkeit der Einzelkomponenten. Besonders günstige Mischungspartner sind z.B. die folgenden Verbindungen:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)pyrimidin-4-yloxy]-phenyl}-3-methoxyacrylat; Methyl-(E)-methoximino-[alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, Chloethocarb, Chlorethoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Didiphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron ,Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Vorzugsweise erhalten die Wirkstoffkombinationen den Wirkstoff zu 0,1 bis 99,9 %, insbesondere zu 1 bis 75 %, besonders bevorzugt 5 bis 50 %, wobei der Rest zu 100 % durch einen oder mehrere der obengenannten Mischungspartner ausgefüllt wird.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentration in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75%.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-% bezogen auf das zu schützende Material.

Die Herstellung und die Verwendung von erfindungsgemäßen Wirkstoffen werden durch die folgenden Beispiele veranschaulicht.

### Herstellungsbeispiele:

### Beispiel 1

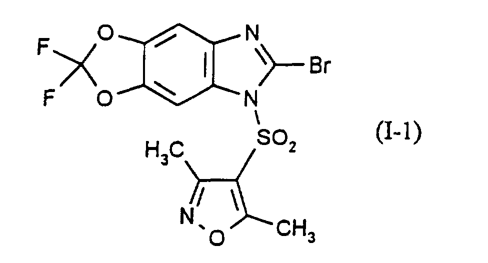

### Verfahren (a)

Ein Gemisch aus 1,4 g (5 mmol) 2-Brom-6,6-difluor-[1,3]dioxolo[4,5f]benzimidazol und 30 ml absolutem Tetrahydrofuran wird bei Raumtemperatur unter Rühren mit 0,2 g (5 mmol) Natriumhydrid (60%ig) versetzt und danach 30 Minuten bei Raumtemperatur gerührt. Anschließend fügt man 1,0 g (5,5 mmol) 3,5-Dimethylisoxazol-4-sulfonylchlorid hinzu und rührt weitere 3 Stunden bei Raumtemperatur. Zur Aufarbeitung wird das Reaktionsgemisch in 100 ml Wasser gegossen. Das entstehende Gemisch wird zweimal mit je 50 ml Essigsäureethylester extrahiert. Die vereinigten organischen Phasen werden über Natriumsulfat getrocknet und unter vermindertem Druck eingeengt. Der verbleibende Rückstand wird mit Methylenchlorid als Laufmittel an Kieselgel chromatographiert. Man erhält auf diese Weise 1,2 g (75% der Theorie) an 1-(3,5-Dimethylisoxazol-4-sulfonyl)-2-brom-6,6-difluor-[1,3]-dioxolo-[4,5f]benzimidazol in Form eines farblosen Feststoffes vom Schmelzpunkt 130-134°C.

### Herstellung von Vorprodukten

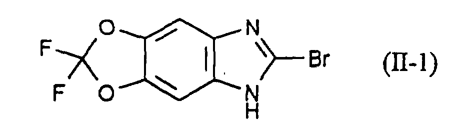

### Verfahren (b)

Zu einer Suspension von 0,48 g (12 mmol) 60%igem Natriumhydrid in 20 ml absolutem Dimethylformamid gibt man 2,0 g (10 mmol) 6,6-Difluor-[1,3]dioxolo[4,5-f]benzimidazol, rührt 30 Minuten bei 20°C, gibt dann 2,2 g (12 mmol) N-Bromsuccinimid zu und rührt weitere 30 Minuten bei gleicher Temperatur. Die Mischung wird auf 250 g Eis gegeben, mit Eisessig auf pH = 4 eingestellt und dreimal mit je 100 ml Essigsäureethylester extrahiert. Die vereinigten organischen Extrakte werden über Natriumsulfat getrocknet und im Vakuum eingeengt. Der verbleibende Rückstand wird mit 50 ml Wasser verrührt und der erhaltene Niederschlag wird abfiltriert und getrocknet.

Man erhält 2,3 g (83% der Theorie) 2-Brom-6,6-difluor-[1,3]dioxolo[4,5-f]benzimidazol als weißen Feststoff mit einem Schmelzbereich von 160 bis 164°C.

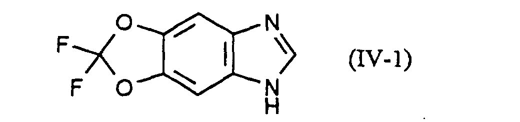

### Verfahren (d)

Zu 37,6 g (0,2 mol) 5,6-Diamino-2,2-difluorbenzodioxol gibt man 80 ml 98%ige Ameisensäure und erhitzt 4 Stunden unter Rückfluß. Nach dem Abkühlen wird mit 2 N Natronlauge alkalisch gestellt. Der Niederschlag wird abfiltriert, mit 200 ml Wasser gewaschen und getrocknet.

Man erhält 38,1 g (95% der Theorie) an 6,6-Difluor[1,3]dioxolo[4,5-f]-benzimidazol in Form eines Feststoffes.

Nach den zuvor angegebenen Methoden werden auch die in der folgenden Tabelle aufgeführten Stoffe hergestellt.

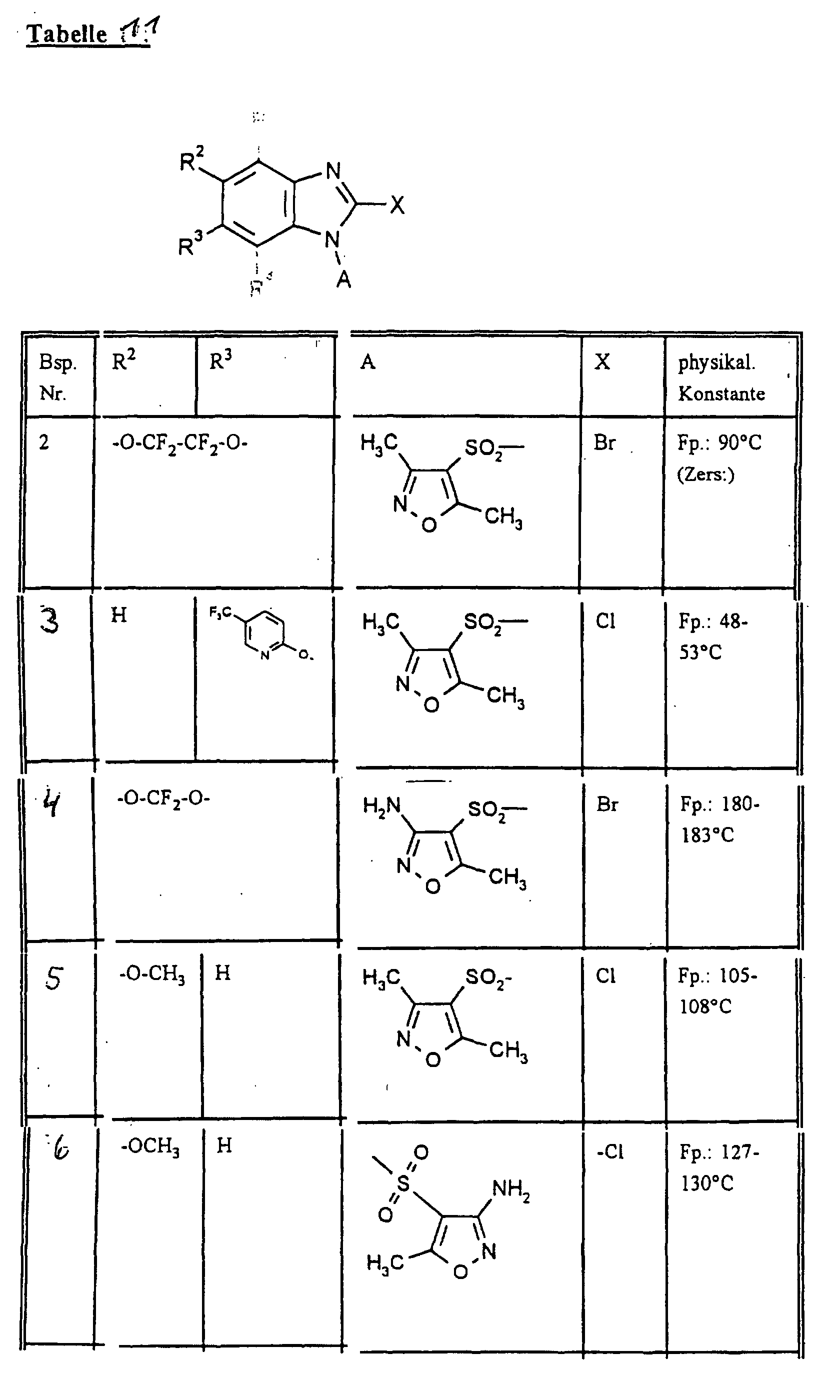

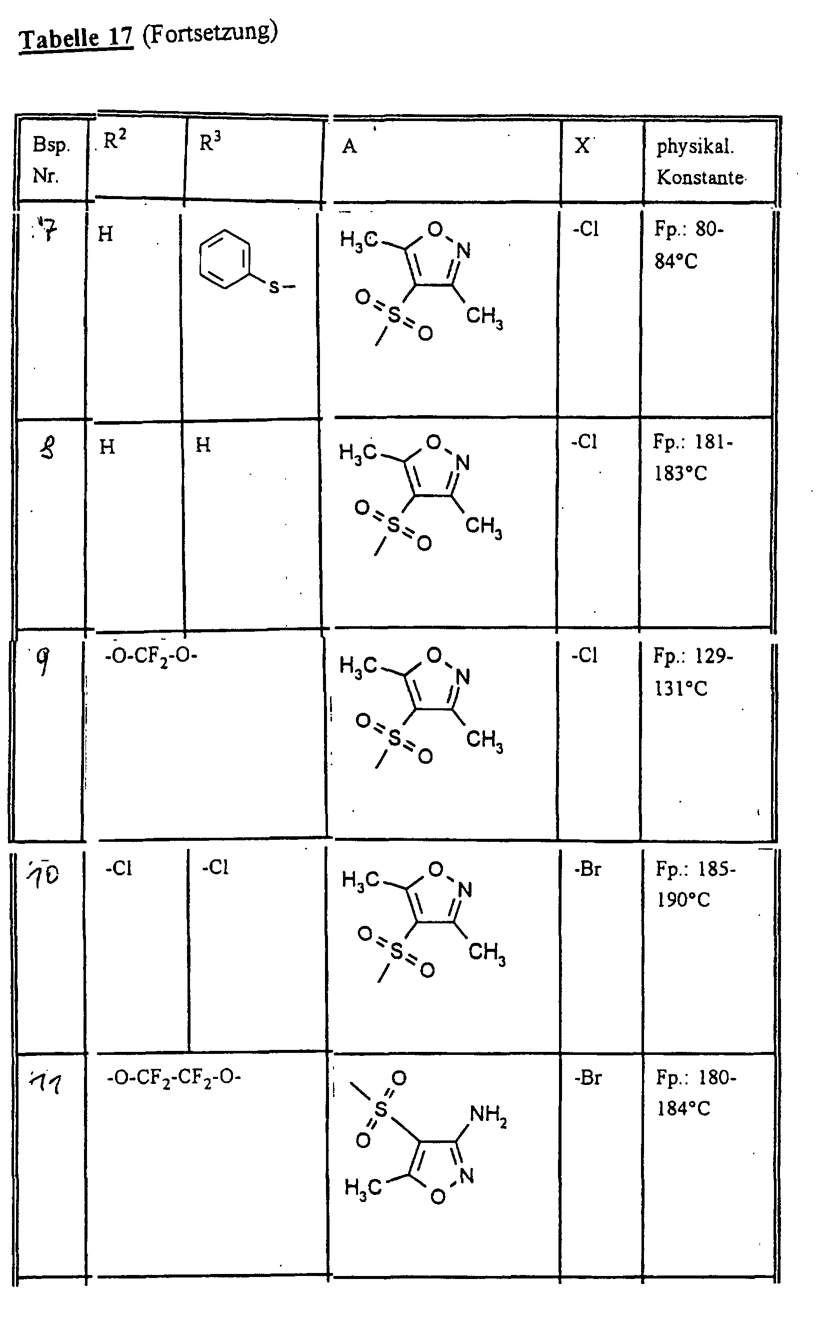

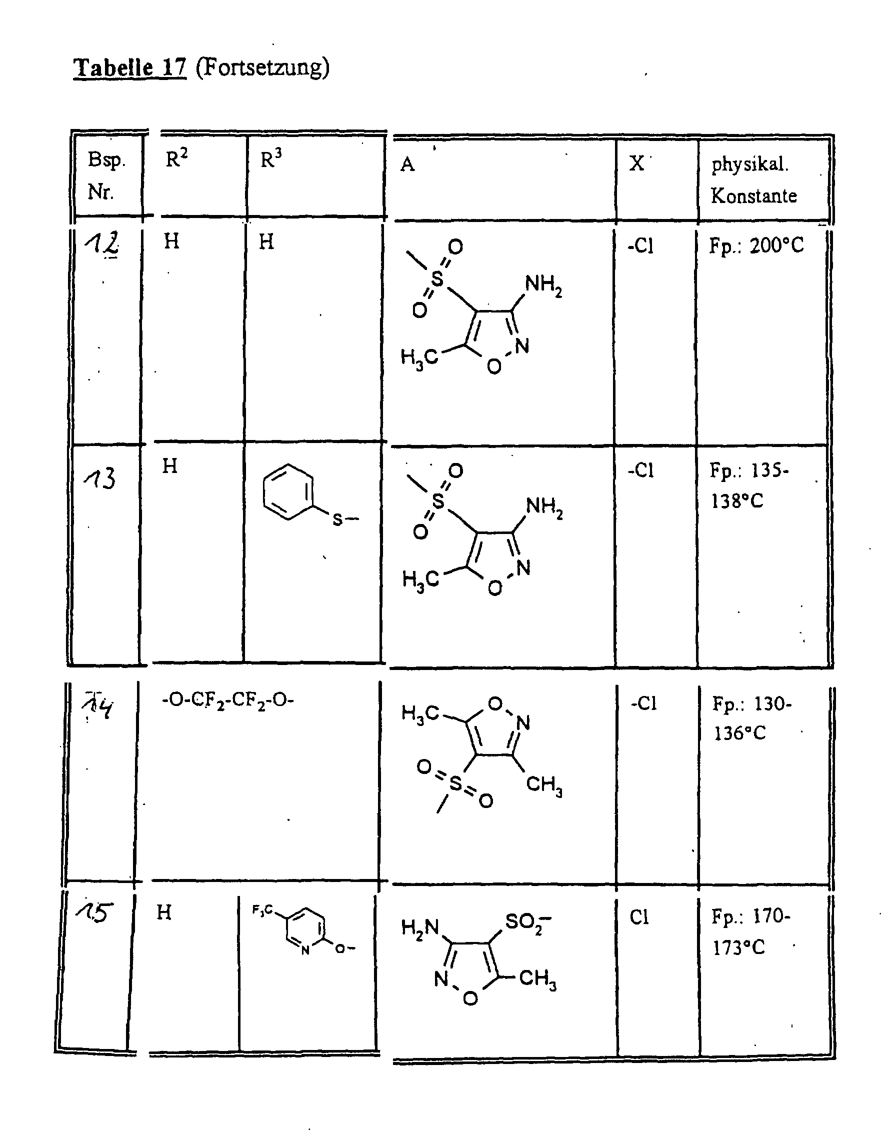

### Beispiel 16

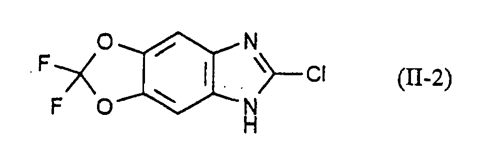

### Verfahren (c)

In eine Lösung von 2,8 g (10 mmol) 2-Brom-6,6-difluor-[1,3]dioxolo[4,5-f]benzimidazol in 30 ml Dimethylformamid wird 2 Stunden lang bei 120°C Chlorwasserstoffgas eingeleitet. Die Mischung wird auf 200 g Eis/Wasser gegeben, dreimal mit je 80 ml Essigsäureethylester extrahiert. Die vereinigten organischen Extrakte werden über Natriumsulfat getrocknet und unter vermindertem Druck eingeengt. Der Rückstand wird mit Diethylether an Kieselgel chromatographiert. Man erhält 0,5 g (21,5% der Theorie) 2-Chlor-6,6-difluor-[1,3]dioxolo[4,5-f]benzimidazol als weißen Feststoff vom Schmelzpunkt >220°C.

### Beispiel 17

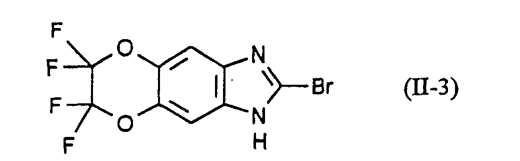

Nach der im Beispiel 1 beschriebenen Methode wird auch 2-Brom-6,6,7,7-tetrafluor-[1,4]dioxino[2,3-fl-benzimidazol hergestellt.
Schmelzpunkt: 170-174°C

### Beispiel 18

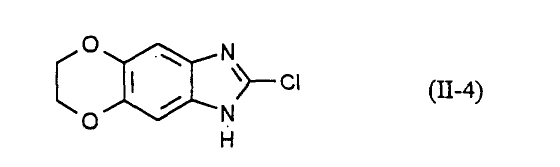

Nach der im Beispiel 1 angegebenen Methode wird auch 2-Chlor-[1,4]-dioxino-[2,4-f]-benzimidazol hergestellt.
Schmelzpunkt: 150°C

### Beispiel 19

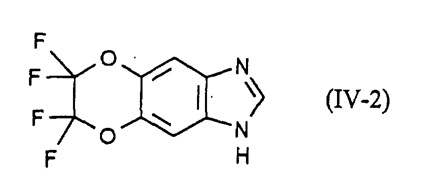

Nach der im Beispiel 1 angegebenen Methode wird auch 6,6,7,7-Tetrafluor-[1,4]-dioxino[2,3-f]benzimidazol hergestellt.
Schmelzpunkt: 70-74°C

### Verwendungsbeipiele

### Beispiel A

### Phytophthora-Test (Tomate) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

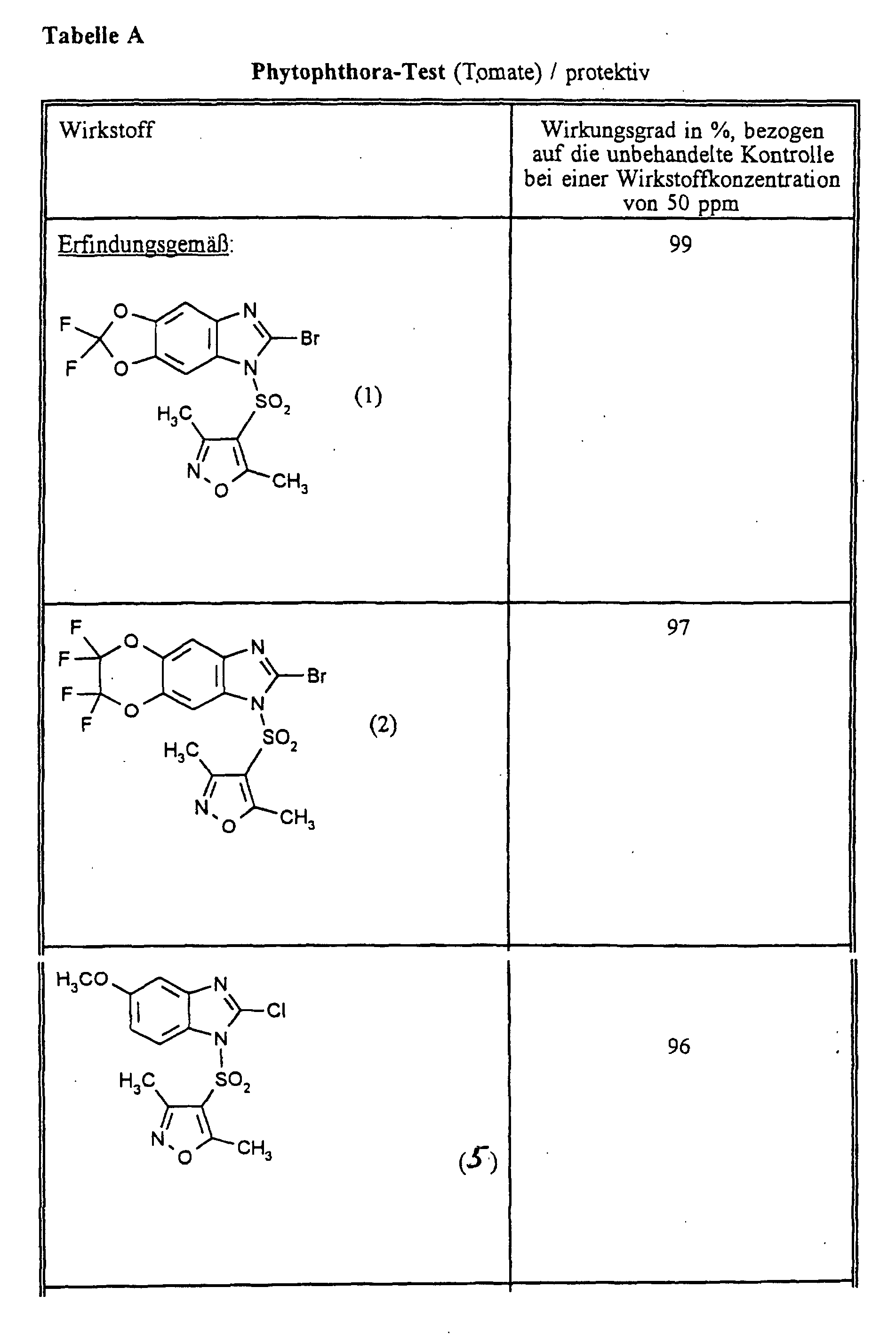

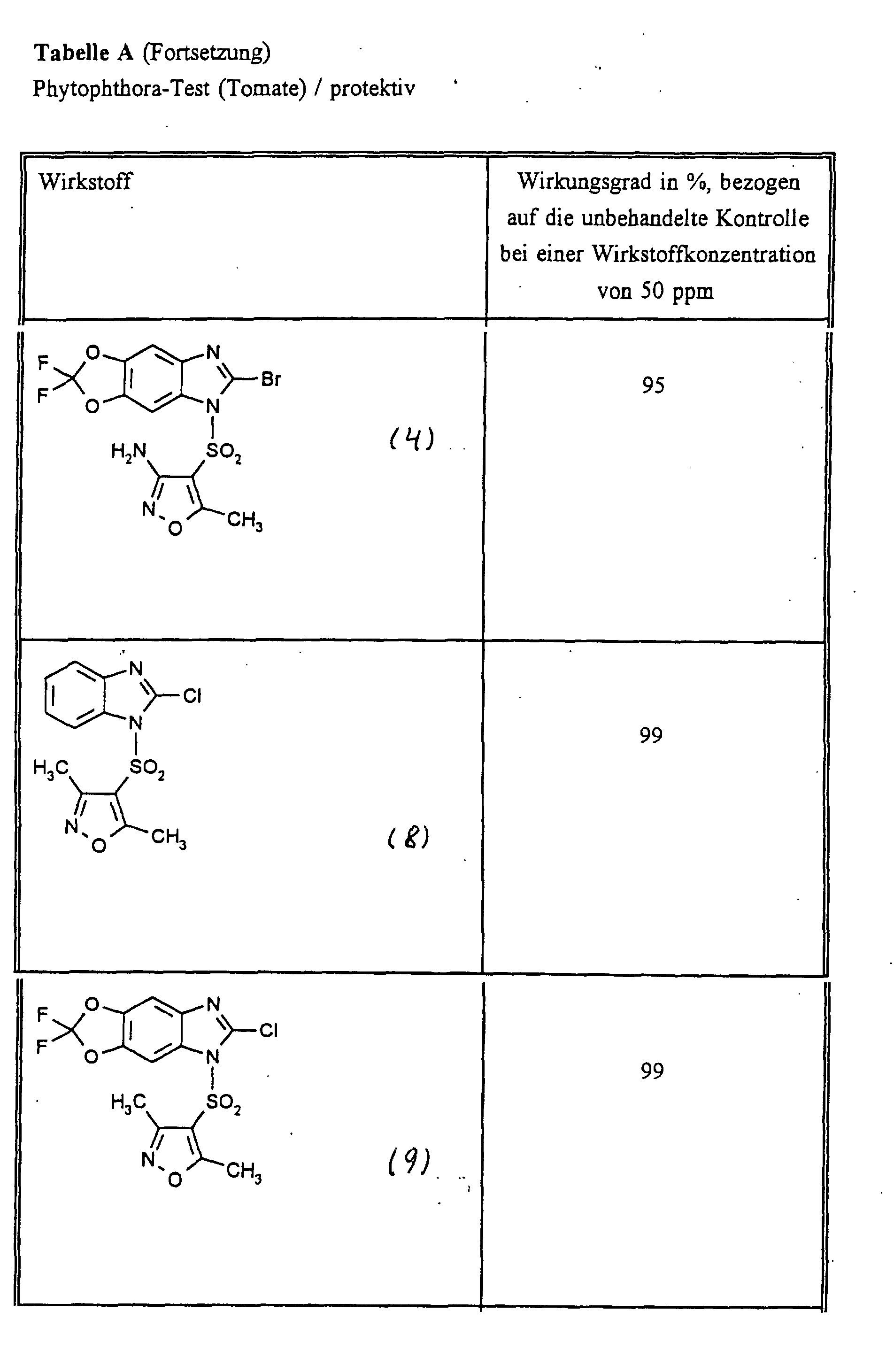

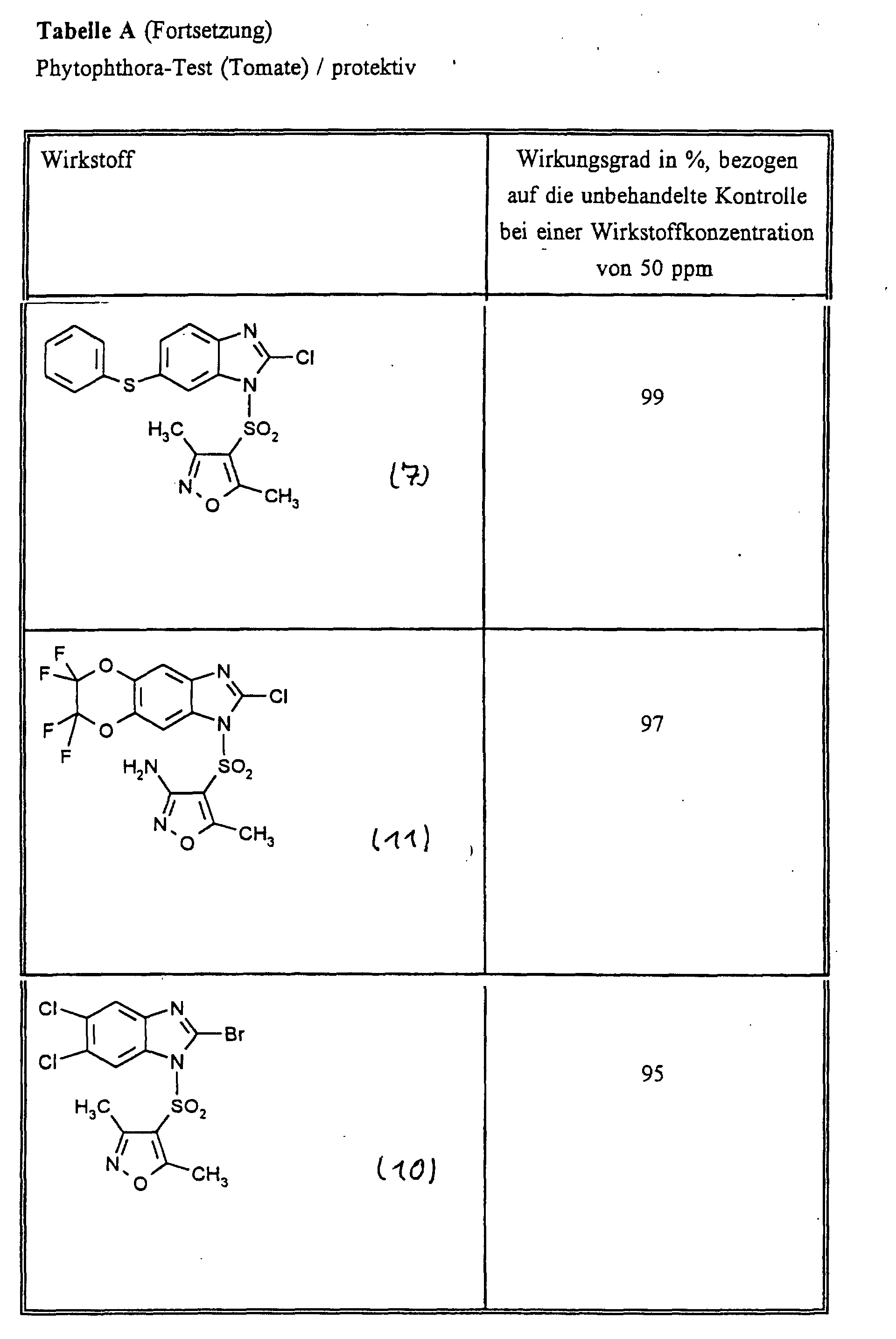

### Beispiel B

### Plasmopara-Test (Reben) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Feuchtkammer gestellt.

6 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

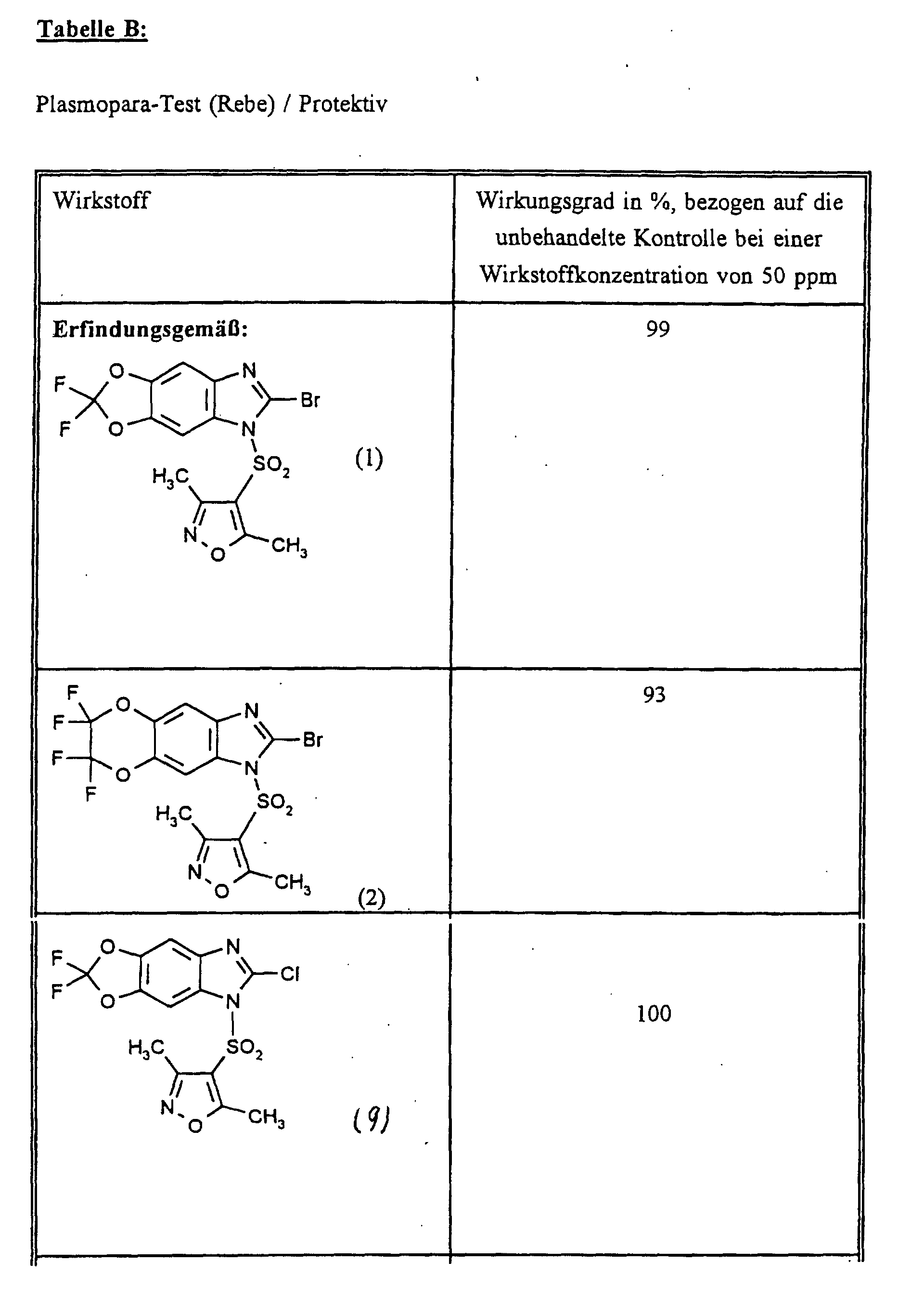

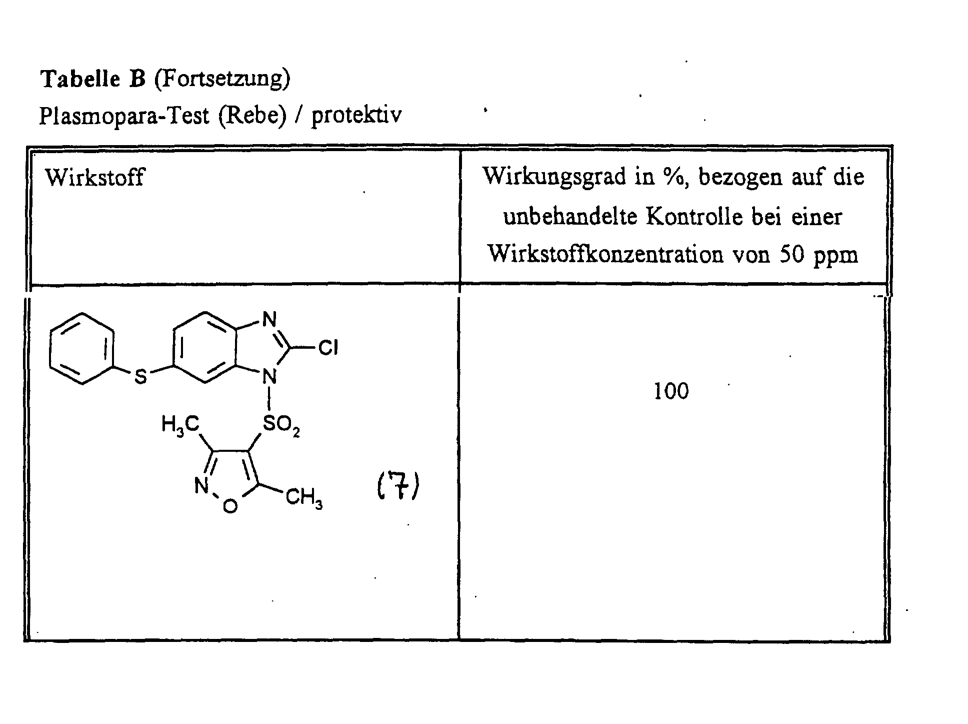

## Patentansprüche

1. Halogenbenzimidazole der Formel 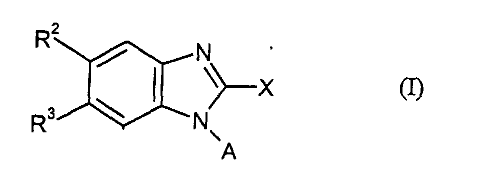 in welcher
R² für Wasserstoff, Fluor, Chlor oder Methoxy steht,
R³ für Wasserstoff, Fluor, Chlor oder für -Z-R⁵ steht,
worin
Z für Sauerstoff oder Schwefel steht und
R⁵ für Phenyl steht, das einfach bis dreifach, gleichartig oder verschieden substituiert sein kann durch Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Difluormethylsulfinyl und/oder Trifluormethylsulfonyl, oder
R⁵ für Pyrrolyl, Furyl, Thienyl, Pyrazolyl, Imidazolyl, Thiazolyl, Isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridinyl, Pyrimidinyl, Pyrazinyl oder Pyridazinyl steht, wobei jeder dieser Reste einfach oder zweifach, gleichartig oder verschieden substituiert sein kann durch Fluor, Chlor, Methyl, Methoxy, Trifluormethyl, und/oder Trifluormethoxy,
oder
R² und R³ gemeinsam für die Gruppierungen -CF₂-O-CF₂-, -O-CF₂-O-, -O-CF₂-CHF-O-, -O-CHF-CHF-O-, -O-CF₂-CF₂-O-, -O-CF₂-CFCl-Ooder -O-CFCl-CFCl-O- stehen,
X für Fluor, Chlor oder Brom steht und
A für eine Gruppierung -SO₂-R⁸ steht, worin
R⁸ für Isoxazolyl steht, wobei dieser Rest einfach oder zweifach, gleichartig oder verschieden substituiert sein kann durch Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylanimo, Ethylamino, n-oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroxyminomethyl, Hydroximinoethyl, Methoxyiminomethyl, Ethoxyiminomethyl, Methoxyiminoethyl und/oder Ethoxyiminoethyl.

2. Halogenbenzimidazol gemäß Anspruch 1, **gekennzeichnet durch** die Formel

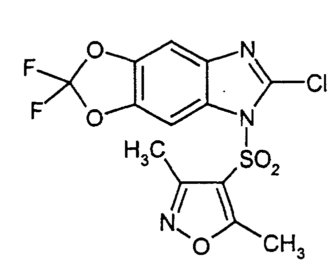

3. Verfahren zur Herstellung von Halogenbenzimidazolen der Formel (I) gemäß Anspruch 1, **dadurch gekennzeichnet, dass** man
a) Benzimidazole der Formel 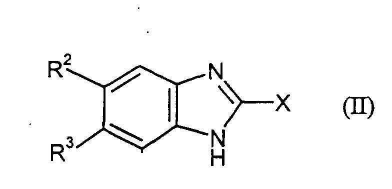
in welcher
R², R³ und X die oben angegebenen Bedeutungen haben,
mit Halogeniden der Formel
A-X¹ (III)
in welcher
A die oben angegebene Bedeutung hat und
X¹ für Chlor oder Brom steht,
gegebenenfalls in Gegenwart eines Säurebindemittels und gegebenenfalls in Gegenwart eines Verdünnungsmittels umsetzt.

4. Mikrobizide Mittel, **gekennzeichnet durch** einen Gehalt an mindestens einem Halogenbenzidazol der Formel (I) gemäß Anspruch 1 sowie **durch** einen Gehalt.an Streckmitteln und/oder oberflächenaktiven Stoffen.

5. Verwendung von Halogenbenzimidazolen der Formel (I) gemäß Anspruch 1 als Mirkobizide im Pflanzenschutz und im Materialschutz.

6. Verfahren zur Bekämpfung von unerwünschten Mikroorganismen im Pflanzenschutz und im Materialschutz, **dadurch gekennzeichnet, dass** man Halogenbenzimidazole der Formel (I) gemäß Anspruch 1 auf die Mikroorganismen und/oder deren Lebensraum ausbringt.

7. Verfahren zur Herstellung von mikrobiziden Mitteln, **dadurch gekennzeichnet, dass** man Halgoenbenzimidazole der Formel (I) gemäß Anspruch 1 mit Streckmitteln und/oder oberflächenaktiven Stoffen vermischt.

## Claims

1. Halogenobenzimidazoles of the formula 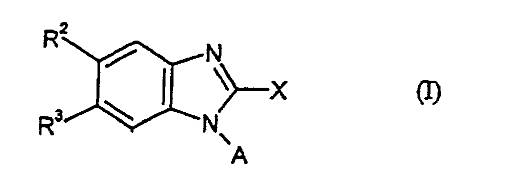 in which
R² represents hydrogen, fluorine, chlorine or methoxy,
R³ represents hydrogen, fluorine, chlorine or―Z-R⁵,
where
Z represents oxygen or sulphur and
R⁵ represents phenyl which can be monosubstituted to trisubstituted by identical or different substituents from the series consisting of fluorine, chlorine, bromine, cyano, nitro, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl, ethylsulphonyl, trifluoromethyl, difluoromethoxy, trifluoromethoxy, difluoromethylthio, trifluoromethylthio, difluoromethylsulphinyl and/or trifluoromethylsulphonyl, or
R⁵ represents pyrrolyl, furyl, thienyl, pyrazolyl, imidazolyl, thiazolyl, isothiazolyl, oxazolyl, isoxazolyl, oxadiazolyl, thiadiazolyl, 1,2,3-triazinyl, 1,2,4-triazinyl, 1,3,5-triazinyl, pyridinyl, pyrimidinyl, pyrazinyl or pyridazinyl, it being possible for each of these radicals to be monosubstituted or disubstituted by identical or different substituents from the series consisting of fluorine, chlorine, methyl, methoxy, trifluoromethyl and/or trifluoromethoxy,
or
R² and R³ together represent the groups -CF₂-O-CF₂-, -O-CF₂-O-, -O-CF₂-CHF-O-, -O-CHF-CHF-O-, -O-CF₂-CF₂-O-, -O-CF₂-CFCl-O-or -O-CFCl-CFCl-O-,
X represents fluorine, chlorine or bromine and
A represents a group -SO₂-R⁸ where
R⁸ represents isoxazolyl, it being possible for this radical to be monosubstituted or disubstituted by identical or different substituents from the series consisting of fluorine, chlorine, bromine, cyano, nitro, amino, hydroxyl, formyl, carboxy, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl, ethylsulphonyl, trifluoromethyl, difluoromethoxy, trifluoromethoxy, difluoromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroxyiminomethyl, hydroxyiminoethyl, methoxyiminomethyl, ethoxyiminomethyl, methoxyiminoethyl and/or ethoxyiminoethyl.

2. Halogenobenzimidazole according to Claim 1, **characterized by** the formula

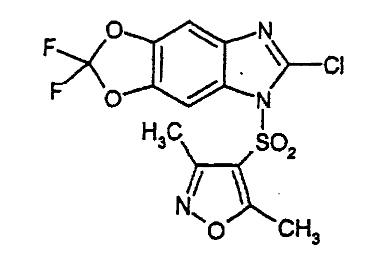

3. Process for the preparation of halogenobenzimidazoles of the formula (I) according to Claim 1, **characterized in that**
(a) benzimidazoles of the formula 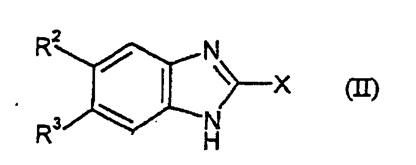 in which
R², R³ and X have the abovementioned meanings,
are reacted with halides of the formula
A-X¹ (III)
in which
A has the abovementioned meaning and
X¹ represents chlorine or bromine,
if appropriate in the presence of an acid-binding agent and if appropriate in the presence of a diluent.

4. Microbicidal compositions, **characterized in that** they comprise at least one halogenobenzimidazole of the formula (I) according to Claim 1 and extenders and/or surfactants.

5. Use of halogenobenzimidazoles of the formula (I) according to Claim 1 as microbicides in crop protection and in the protection of materials.

6. Method of controlling undesirable microorganisms in crop protection and in the protection of materials, **characterized in that** halogenobenzimidazoles of the formula (I) according to Claim 1 are applied to the microorganisms and/or their environment.

7. Process for the preparation of microbicidal compositions, **characterized in that** halogenobenzimidazoles of the formula (I) according to Claim 1 are mixed with extenders and/or surfactants.

## Revendications

1. Halogénobenzimidazoles de la formule : 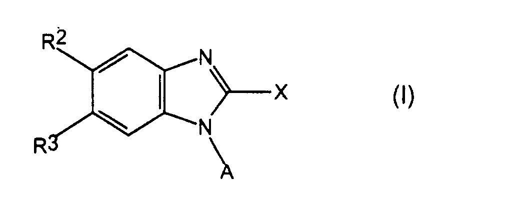 dans laquelle :
R² représente l'atome d'hydrogène, de fluor, de chlore ou le radical méthoxy,
R³ représente l'atome d'hydrogène, de fluor, de chlore ou un radical -Z-R⁵,
où
Z représente l'atome d'oxygène ou de soufre, et
R⁵ représente le radical phényle, qui peut être substitué une à trois fois, de manière identique ou différente, par l'atome de fluor, de chlore, de brome, le radical cyano, nitro, méthyle, éthyle, n- ou i-propyle, n-, i-, s- ou t-butyle, méthoxy, éthoxy, n-ou i-propoxy, méthylthio, éthylthio, n- ou i-propylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, difluorométhylsulfinyle et/ou trifluorométhylsulfonyle, ou
R⁵ représente le radical pyrrolyle, furyle, thiényle, pyrazolyle, imidazolyle, thiazolyle, isothiazolyle, oxazolyle, isoxazolyle, oxadiazolyle, thiadiazolyle, 1,2,3-triazinyle, 1,2,4-triazinyle, 1,3,5-triazinyle, pyridinyle, pyrimidinyle, pyrazinyle ou pyridazinyle, où chacun de ces restes peut être substitué une ou deux fois, de manière identique ou différente, par l'atome de fluor, de chlore, le radical méthyle, méthoxy, trifluorométhyle et/ou trifluorométhoxy, ou
R² et R³ représentent ensemble, le groupement -CF₂-O-CF₂-, -O-CF₂-O-, -O-CF₂-CHF-O-, -O-CHF-CHF-O-, -O-CF₂-CF₂-O-, -O-CF₂-CFCl-O- ou -O-CFCl-CFCl-O- ;
X représente l'atome de fluor, de chlore ou de brome ;
A représente un groupement -SO₂-R⁸, où
R⁸ représente le radical isoxazolyle, où ce reste peut être substitué une ou deux fois, de manière identique ou différente, par l'atome de fluor, de chlore, de brome, le radical cyano, nitro, amino, hydroxyle, formyle, carboxyle, carbamoyle, thiocarbamoyle, méthyle, éthyle, n- ou i-propyle, n-, i-, s- ou t-butyle, méthoxy, éthoxy, n- ou i-propoxy, méthylthio, éthylthio, n- ou i-propylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle ou trifluorométhylsulfonyle, méthylamino, éthylamino, n- ou i-propylamino, diméthylamino, diéthylamino, acétyle, propionyle, acétyloxy, méthoxycarbonyle, éthoxycarbonyle, méthylsulfonyloxy, éthylsulfonyloxy, hydroxyiminométhyle, hydroxyiminoéthyle, méthoxyiminométhyle, éthoxyiminométhyle, méthoxyinoéthyle et/ou éthoxyiminoéthyle.

2. Halogénobenzimidazole suivant la revndication 1, **caractérisé par** la formule :

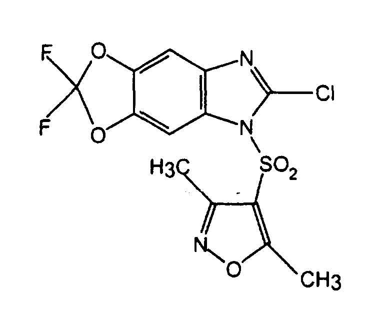

3. Procédé de préparation d'halogénobenzimidazoles de la formule (I) suivant la revendication 1, **caractérisé en ce que** l'on :
a) fait réagir les benzimidazoles de la formule : 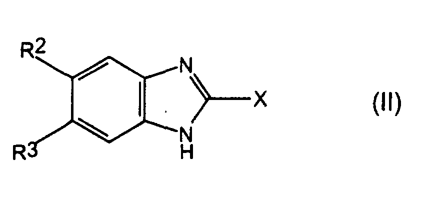
dans laquelle R², R³ et X ont les significations indiquées ci-dessus,
avec des halogénures de la formule :
A-X¹ (III)
dans laquelle:
A a la signification indiquée ci-dessus, et
X¹ représente l'atome de chlore ou de brome,
le cas échéant en présence d'un agent liant les acides et le cas échéant, en présence d'un agent de dilution.

4. Agent microbicide, **caractérisé par** une teneur en au moins un halogénobenzimidazole de la formule (I) suivant la revendication 1, ainsi que par une teneur en un diluant et/ou tensioactif.

5. Utilisation des halogénobenzimidazoles de la formule (I) suivant la revendication 1, comme microbicide dans la protection des végétaux et dans la protection des matériaux.

6. Procédé pour lutter contre les microorganismes non souhaités dans la protection des végétaux et dans la protection des matériaux, **caractérisé en ce que** l'on applique les halogénobenzimidazoles de la formule (I) suivant la revendication 1, sur les microorganismes et/ou leur biotope.

7. Procédé de préparation d'agents microbicides, **caractérisé en ce que** l'on mélange les halogénobenzimidazoles de la formule (I) suivant la revendication 1, avec des diluants et/ou tensioactifs.
